# EUROPEAN PATENT SPECIFICATION

(11) **EP 3 247 335 B1**
(45) Date of publication and mention of the grant of the patent: **08.07.2020**
(21) Application number: 16705599.5
(22) Date of filing: 25.01.2016
(51) Int. Cl.: A61K 9/14, A61K 31/07, A61K 31/355, A61K 31/375, A61K 31/715, A61K 33/26, A61K 35/741, A61K 36/06, A61K 45/06, A61K 47/44, A61P 3/02, A61P 7/00, A61P 43/00, A61K 36/899, A61K 47/10, A61K 9/16, A61K 9/00, A61K 36/30, A61K 36/55, A61K 36/63

(54) **FLUID COMPOSITION CONTAINING A SOURCE OF IRON IN NON-IONIC FORM AND METHODS OF USE**
FLUIDZUSAMMENSETZUNG MIT EINER QUELLE VON EISEN IN NICHTIONISCHER FORM UND VERFAHREN ZUR VERWENDUNG
COMPOSITION DE FLUIDE CONTENANT UNE SOURCE DE FER SOUS FORME NON IONIQUE ET PROCÉDÉS D'UTILISATION ASSOCIÉS

(30) Priority: 25.01.2015 PL 41106215
(43) Date of publication of application: 29.11.2017
(73) Proprietor: Wrzosek, Artur, 05-532 Tomice (PL); Klys, Piotr, 05-500 Stara Iwiczna (PL); Cieciara, Mariusz, 05-520 Konstancin-Jeziorna (PL)
(72) Inventor: Wrzosek, Artur, 05-532 Tomice (PL); Klys, Piotr, 05-500 Stara Iwiczna (PL); Cieciara, Mariusz, 05-520 Konstancin-Jeziorna (PL)
(74) Representative: Padée, Grazyna
(86) International application number: PCT/PL2016/000008
(87) International publication number: WO 2016/118028

(56) References cited:
- WO-A1-2010/146155
- AU-A1- 2013 206 429
- US-A1- 2007 065 521
- RICHARD C. THEUER: "Iron-Fortified Infant Cereals", FOOD REVIEWS INTERNATIONAL, vol. 24, no. 3, 30 May 2008 (2008-05-30), pages 277-310, XP055259518, Philadelphia, USA ISSN: 8755-9129, DOI: 10.1080/87559120801926260
- MURRAY D. PENNELL ET AL: "FACTORS AFFECTING THE RELATIVE BIOLOGICAL VALUE OF FOOD GRADE ELEMENTAL IRON POWDERS FOR RATS AND HUMANS", JOURNAL OF FOOD SCIENCE, vol. 40, no. 4, 1 July 1975 (1975-07-01), pages 879-883, XP055598935, US ISSN: 0022-1147, DOI: 10.1111/j.1365-2621.1975.tb00580.x
- VERMA RS ET AL: "Effect of storage in flour and of particle size on the bioavailability of elemental iron powders for rats and humans.", J ASSOC OFF ANAL CHEM. 1977 JUL;60(4):759-65., 1 January 1977 (1977-01-01), XP055598942,

## Description

The invention relates to a fluid composition containing a source of iron in non-ionic form with strictly defined particle size. The composition may be used as a pharmaceutical preparation or a dietary supplement.

Iron is one of the most important chemical elements necessary for normal functioning of the organism. This microelement is a main component of haemoglobin, which transports oxygen. It controls the process of proper production of red blood cells and haemoglobin, deciding on correct oxygen transport in the organism. Iron also plays an important role in the proper functioning of the immune system, maintaining proper cognitive functions, and in the cell division process. The daily demand for iron depends on age, sex and state of health. The demand is different in adults, children, pregnant women, breast-feeding women, as well as in persons after surgical interventions resulting in large blood loss. It is assumed that the average daily demand for iron amounts to 10-20 mg, according to Commission Directive 2008/100/EC of 28 November 2008. The RDA for iron is 14 mg. Disturbance in iron management may lead to numerous adverse changes in proper functioning of the organism, of which anaemia is the most important one. It is the most frequent and the most globally widespread anaemia type, manifesting itself by a too low haemoglobin level and a small number of red blood cells.

The availability of iron from food products is relatively low and ranges from small amounts to 20% maximum. Therefore, many persons use iron-containing preparations, but - as is shown by scientific studies - its availability in the form of soluble salts is not high either, and it usually does not exceed 25%.

Because of the difficult parameters of iron availability, the element is used in medicine in various chemical forms, adapted to specific pharmaceutical forms and routes of their administration. The most popular and most frequently used forms include soluble iron salts. They include both inorganic and organic compounds, such as: iron(II) sulfate(VI), iron(III) chloride, iron(III) diphosphate(V), iron(II) fumarate or iron(II) gluconate. Also, preparations containing elemental iron are known. The form and percentage of iron in a soluble salt determine the absorption of the element, as well as its subsequent biological activity. Considering the high molar mass and low percentage of iron (10-36%) in its salts, and their hindered bioavailability, in order to obtain the desired therapeutic effect, application of high doses of iron salts is necessary, leading to an increase in side effects in the gastrointestinal system. Moreover, application of high doses of soluble salts increases the risk of poisoning or overdose of iron-containing preparations, and such events require a physician's intervention. The oxidation state of iron included into the composition of the soluble salt is also of high importance - Fe³⁺ ions exhibit a significantly lower bioavailability than Fe²⁺ ions (the mechanism of iron transport is connected with DMT (Divalent Metal Transporter)).

Some of these problems are solved by using elemental iron. Elemental iron is transformed into ionised form not earlier than in the digestive tract, under the influence of gastric juice and the hydrochloric acid it contains. Elemental iron is characterised by a high iron content exceeding 98% and low molar mass, enabling use of its minimum doses in order to satisfy the daily demand for iron, when compared to those of an iron(II) salt.

A vast range of pharmaceutical preparations and dietary supplements, complementing iron deficiencies or satisfying an increased demand for iron, is known. These preparations are in the form of tablets, effervescent tablets, capsules and sachets, as well as in fluid form, such as syrup or tonic.

An iron deficiency supplementation plan should have linear characteristics, adapted to individual needs. The optimal solution is constituted by a preparation which allows for dosing iron per kilogram of body weight. Preparations in the form of tablets, effervescent tablets, capsules and sachets are characterised by threshold dosing, and they do not offer any possibility of individual adjustment of the applied iron dose in a manner involving a simple calculation. Products having forms of a syrup or an oral solution do offer such a possibility; however, their use is connected with a dark staining of the teeth. Depending on the iron source used, the effect of a metallic taste in the mouth also occurs, perceived as a disadvantageous impression.

Known preparations containing elemental iron take the form of tablets or capsules. Examples of fluid compositions containing non-ionic iron sources in the form of elemental iron, based on water as a vehicle, are also known. Such compositions, considering the oxidative properties of water, require the use of a broad range of additional substances, conservatives and stabilisers such as: EDTA, tert-butylhydroquinone (TBHQ), methylparaben or propylparaben. The necessity to use these substances and their quantitative limits in medicinal or food products limits or even precludes the use of such a composition for supplementation of iron deficiencies in children and infants. Another problem consists in the fact that as a result of the oxidative action of water, a ternary mixture may form, containing iron in the unchanged form, as well as iron ions with various oxidation states (Fe²⁺, Fe³⁺), and the proportions of these three forms in the mixture are unknown.

An example of an oral veterinary composition containing elemental iron for use in infants of mammals other than human, disclosed in Patent Application No. WO 2010/146155A1, is also known. This application pertains to a preparation having a form of a gel with a high content of micronized elemental iron, with oil as a vehicle. The iron content in the preparation may range from 5 to 30%, preferably from 10 to 25%, most preferably 20%. The preferable particle size of elemental iron was defined in the range from 3-7 µm.

Such a small particle size and, consequently, the high surface area of the active substance resulting from it leads to an increase in the rate of transformation of elemental iron to Fe²⁺ ions. This results in reaching a high concentration of iron in the ionic form in the organism over a short time. The effect of the rapid increase in ion concentration is additionally enhanced by the high content of iron in the preparation. The high content of elemental iron in the preparation, connected with the small particle size, might seem highly preferable for a person skilled in the art in the context of supplementation of iron deficiencies. Larger particles of elemental iron with a smaller surface area, having a lower rate of transformation to Fe²⁺ ions, might then seems less preferable in the process of supplementation of iron deficiencies, due to the lower concentration achieved in the same time. However, a high concentration of iron ions is not tantamount to its high availability, as the latter is limited by several biochemical processes occurring in the organism. The level of iron in the organism is regulated by duodenum, as it is here that epithelial cells, enterocytes, are responsible for absorption of iron from food. The best-known mechanism of iron transport is that connected with DMT (Divalent Metal Transporter). The Divalent Metal Transporter (DMT1) transports iron cations and other metals at oxidation state II from the intestine lumen into the enterocytes. In this stage, the presence of auxiliary proteins is necessary, which reduce the iron present in food. It is only then that the iron present in the enterocytes is transported to the blood vessels. A high concentration of Fe²⁺ ions obtained over a short period, resulting from the use of particles with small sizes and a large surface area for the reaction, connected with the biochemical limitations of the iron assimilation process, leads to the formation of an excess of ions not used by the organism. The unused excess of ions present at a high concentration may lead to a range of microbiological equilibrium disturbances of bacterial flora in the intestine of the organism. Numerous studies, carried out using both animal and human models, confirm the argument of the influence of iron supplementation on the immune system. As proven in the paper entitled Iron and Infection EUGENE D. WEINBERG, MICROBIOLOGICAL REVIEWS, Mar. 1978, Vol. 42, No. L, p. 45-66*,* infants subjected to an iron load exceeding the absorptive capacity of the organism exhibit a distinctly lowered immunity to bacterial infections. Similar associations were obtained in studies on infants wherein a relation between an oversupply of iron and occurrence frequency of sepsis induced by Gram-negative bacteria was statistically proven. Barry, D. M. J., and A. W. Reeve. 1977. Increased incidence of gram-negative neonatal sepsis with intramuscular iron administration. Pediatrica 10:908-912*.*

It was also undoubtedly proven that iron is a factor limiting the growth and pathologic activity of some microorganisms colonising the human digestive system. Followed by continual competition for a source of iron between the intestinal iron transport system and it use by pathogenic microorganisms. As soon as the iron oversupply exceeds the assimilation capacity of the organism, the iron is used by these microorganisms. The redox potential of iron leads to its transformation from the II to III oxidation state, being in turn a driving force for many pathogens, resulting in oxidative stress, and as a consequence - in damage to the structures of DNA, lipids and proteins. Iron in Infection and Immunity James E. Cassatl and Eric P. Skaar Cell Host & Microbe 13, May 15, 2013 a2013 Elsevier Inc. Scientists report that natural immune mechanisms strictly depend on maintaining an environment with a low iron supply - the organism is being aided in this by specialised structures of iron-binding proteins, transferrin and lactoferrin (The Critical Role of Iron in Some Clinical Infections J.J. Bullen, C.G. Ward, H.J. Rogers, Eur. J. Clin. Microbiol. lnfect. Dis., August 1991, p. 613-617*).* When iron becomes available in amounts exceeding the capacity of natural iron capture mechanisms (free ions in an amount above 10⁻¹⁸), the mechanisms are disturbed (Bullen, JJ, Rogers, H.J. and Griffiths, E. (1978) Role of iron in bacterial infection. Curr. Top. Microbiol. Immunol. 80, 1-35*).*

The publication *"*Iron-Fortified Infant Cereals", R.C. Theuer (FOOD REVIEWS INTERNATIONAL, vol. 24, no. 3, 30 May 2008, pages 277310) discloses iron-fortified infant cereals, however no preparation containing elemental iron particles with the mean size distribution of 7-10 µm was described. In this publication two other documents have been cited (Murray D. Pennell et al: "Factors affecting the relative biological value of food grade elemental iron powders for rats and humans ", Journal of Food Science, vol. 40, no. 4 1 July 1975, pages 879-883*;* Verma Rs et al.: :Effect of storage in flour and of particle size on the bioavailability of elemental iron powders for rats and humans ", PubMed NCBI, J.Assoc Off Anal Chem., 1 January 1977, pages 759-765*),* however those documents only focus on the Relative Biological Values.

AU2013206429 A1 relates to treatment of iron-related conditions with elemental iron, but only carbohydrate complexes of iron was disclosed. US2007065521 disclosed a composition comprising heme iron and/or heme iron polypeptide in combination with ionic iron) and/or chelated iron.

It is of utmost importance that iron preparations are characterised by a very accurate release profile, most preferably a linear profile. Such a profile should allow for obtaining high iron bioavailability, enabling - in connection with an adequately long release time - optimal absorption by the organism, and in consequence - a reduction of a harmful excess of unused ions.

The present invention solves the problem of manufacturing a preparation supplementing iron deficiencies or satisfying an increased demand for iron, without the side effects described above, not generating any risk of poisoning and allowing for proper dosing.

The composition according to the invention has a fluid form and consists of elemental iron with an average particle size in the range from 7 to 10 micrometres in an amount from 0.1% to 15.0% w/w and a vehicle selected from: glycerol, a vegetable and/or fish oil in an amount from 85% to 99.9% w/w, individually or in a mixture. Preferably, elemental iron has a particle size distribution with characteristics of 7µm< D₅₀ < 10µm and D₉₀ < 15 µm.

As an oil, the composition preferably contains: triglycerides of medium-chain fatty acids with a chain length from C6 to C14, maize oil, rapeseed oil, linseed oil, untreated coconut oil, fish-liver oil, cod-liver oil or other fish oils, olive oil, or any mixtures thereof.

Most preferably, the composition contains a mixture of triglycerides of medium-chain fatty acids with a chain length from C6 to C14, most preferably the chains with a length from C8 to C10 constitute not less than 90% w/w of the mixture.

The composition according to the invention may be also enriched with additional components playing an important role in the functioning of the circulatory system and haemopoietic processes, in which iron plays a key role. The additional substances and mechanism of their action determine the degree of suitability in cases of products intended for children and infants and for pregnant women or women planning pregnancy. The components that may enrich the composition include: a mixture of tocopherols (vitamin E) and/or α-tocopherol, organic compounds from the retinoid group (vitamin A) and/or retinol, a group of chemical compounds being derivatives of 2-methyl-1,4-naphthoquinone (vitamin K), the two most important derivatives of which are vitamin K₁ (called phytomenadione, phytonadione, filoquinone) and vitamin K₂ (menaquinone-6, farnoquinone), a group of fat-soluble steroid organic compounds known as D vitamins, the two most important ones from which are: ergocalciferol (vitamin D₂) and cholecalciferol (vitamin D₃), vitamin C (ascorbic acid) and vitamins from the B group, particularly B₆, B₉, and/or its reduced stabilised tetrahydrofolates, e.g. L-5-MTHF, L-5-methyltetrahydrofolate, and B₁₂. Vitamin C aids iron absorption and also plays an important role in the activation of folic acid (vitamin B₉) and in the cellular breathing process, as well as stimulates the maturation of red blood cells. Folic acid plays an important role in haemopoiesis and cooperates with vitamin B₁₂ in regulation of formation and maturation of red blood cells. Vitamin B₁₂ participates in the transformation of folic acid into a biologically active tetrahydrofolate, and its proper level prevents such diseases as pernicious anaemia. Vitamin B₆ participates in the haemoglobin formation process and also increases the immunity of the organism.

The composition according to the invention may also be enriched with substances from the group of natural organic carotenoids, such as: lutein and/or lycopene, and/or zeaxanthin, which, apart from having strong antioxidative and neuroprotective properties, affect iron bioavailability and remove the so-called "coffee effect", being of extremely high importance for adults with an iron deficiency or an increased demand for iron who consume large quantities of coffee.

The composition according to the invention may be also enriched with components from the group of probiotic bacteria: *Lactobacillus rhamnosus, Bifidobacterium animalis, Lactobacillus casei, Lactobacillus bulgaricus,* non-pathogenic pure culture yeast *Saccharomyces boulardii,* or substances from the group of polysaccharides with prebiotic properties: arabinogalactan, inulin. These substances favourably affect the function of the immune system by having an influence on lymphocytes, or they exhibit strong prebiotic activity by aiding the growth of useful intestinal microflora, being a natural barrier to colonisation of the intestine by harmful microorganisms. These components may be useful in the case of application of the preparation for patients highly exposed to pathogenic disturbance of the intestinal microflora: persons with a weakened or compromised immune system, and premature infants, particularly those treated with erythropoietin.

The composition according to the invention contains additional active components in an amount from 0.001 to 10% w/w, depending on the component type.

The invention also includes the application of the aforementioned composition for manufacturing of a preparation for supplementation of iron deficiencies and/or to satisfy an increased demand for iron in mammals, including humans. Because of its unique properties, the composition is intended particularly for administration, in treatment and prophylaxis, to infants, children, pregnant women and women planning pregnancy.

The composition according to the invention is prepared by initial mixing of elemental iron with the first part of the vehicle in an amount from 5 to 30% w/w and then adding the other part of the vehicle, stirring vigorously in order to form a suspension. The additional active components are added to the suspension in the last phase of mixing. The process of mixing and packing the composition is preferably carried out in the protective atmosphere of an inert gas, such as nitrogen, argon, helium.

The vehicle of the composition according to the invention may be mixed in any ratio with another vehicle recommended to use in products intended for children and infants, such as: glycerol, maize oil, olive oil, treated olive oil, generally in order to improve the properties of the product: solubility of the enriching oil- and fat-insoluble components, or to adapt to the taste preferences of individual recipients, as well as to improve flavour. Simultaneously, the vehicle may not have an oxidative character, which might cause an uncontrolled disproportion of iron in the atomic form in relation to iron in the form of ions with various oxidation states (Fe²⁺, Fe³⁺), Use of such a vehicle would lead to a deterioration of iron bioavailability, due to the presence of an ionised form.

The composition according to the invention reduces the possibility of an increased concentration of unused iron ions to arise. This property results from a prolonged rate of conversion of elemental iron to iron ions with the participation of gastric acids, being a consequence of the application of elemental iron with the particle size defined above.

The aforementioned property was confirmed in studies on *in vitro* rate of dissolution of elemental iron in 0.1 M hydrochloric acid carried out by the authors of the invention. Elemental iron with defined characteristics of particle size, by comparison to a particle size median, was used: 8.5 µm, 9.5 µm, and for comparison, 5.2 µm (from the range disclosed in Patent Application No. WO 2010/146155A1). For iron with a particle size median of 5.2 µm, maximum absorbance was already achieved after 15 minutes. For a median of 8.5 µm, maximum absorbance was achieved after 20 minutes, and for 9.5 µm - after 60 min, while in the latter case, a linear dependence of iron release for up to 45 min was obtained.

Basically, the smaller the iron particles, the faster the reaction of its dissolution in hydrochloric acid runs. The authors of the present invention found that there is a very narrow range of particle sizes, for which this process changes from an exponential to linear dependence and then approaches the zero-order characteristics. This border is in a particle size range between 7 and 9.5 micrometres.

The characteristics of dissolution of iron particles in 0.1 M hydrochloric acid translates into the characteristics of iron release from pharmaceutical preparations containing particles with a defined size. A preparation manufactured according to the description of Patent Application No. WO 2010/146155A1 in the form of a gel based on MCT oil and particles with a size of 5.2 micrometres (median D₅₀) exhibits a tendency for exponential release of iron ions. On the other hand, use of particles with a size of 8.5 micrometres in the preparation based on MCT oil leads to linear kinetics, and a maximum concentration is obtained in the time corresponding to gastric passage. As a consequence, a pharmaceutical preparation prepared based on the particle size range defined according to the invention, in connection with the dose determined as the highest possible to assimilate, constitutes a preparation with very efficient availability and minimised side effects connected with the introduction of iron to the organism in an amount exceeding the assimilation capacity, when the excesses of iron ions forming in the intestines may promote growth of non-physiological bacterial flora.

The influence of the reduced concentration of unused iron ions on pathogenic bacteria under conditions simulating the small intestine was shown in another in vitro study carried out by the authors of the invention. The study was carried out using the following strains: E. coli ATCC 25922, Klebsiella pneumoniae BAA-1705, Enterococcus faecalis ATCC 2912 and Staphylococcus aureus with the MRSA ATCC-BAA-36 mechanism. The selected strains represent the flora of the human digestive tract and are of particular importance for newborns and infants, because of the fact that these strains are a main cause of sepsis. As a source of iron, the preparation being the subject of the invention (sample A) and the preparation disclosed in Patent Application No. WO 2010/146155A1 (sample B) were used. Both sources of iron were used in the same amount of 300 µl. In the study, significant differences both in the amount of released iron and iron used by the studied strains after the same incubation time were observed for both samples. After a 24 h incubation period, the following was found: iron consumption higher by 3 times for the standard strain of S. aureus, iron consumption higher by 13 times for the standard strain of E. faecalis, iron consumption higher by 187 times for the standard strain of E. coli ATCC25922, and a slight difference (ca. 1 time) in iron use by the standard strain of K. pneumoniae from sample B in relation to sample A.

In the case of the preparation being the subject of the invention (sample A), 35.6 mg/l was released to an aqueous phase after 48 h, while for the preparation described in Patent Application No. WO 2010/146155A1 (sample B), this concentration amounted to 132.1 mg/l. As in the result of recalculations, it was proven that with an iron concentration equal to 0.026 mg/ml, a 1 log growth of E. coli ATCC 25922 population is observed, and in both studied samples, the number of microorganisms grew to a similar degree after 48 h. However, due to significant differences in the concentrations of released iron and iron absorbed by the microorganisms, a reduced growth of the population of pathogenic bacteria in the case of the preparation being the subject of the invention should be expected under in vivo conditions. Results of the in vitro study show, in an obvious manner, a dependence between the particle size of elemental iron and the release rate of ions, as well as the capacity of bacteria for increased absorption in the case of an increased concentration of unused iron ions. As is indicated by the numerical data, the absorption capacity, and therefore the ability of the pathogenic bacteria population to grow, is considerably limited in the case of an inventive composition with strictly defined particle size of elemental iron.

Thanks to the mentioned properties and possibility to use low doses, the preparation according to the invention, containing elemental iron with a strictly defined particle size, is characterised by infinitesimal toxicity, and the occurrence and intensity of side effects is reduced to a minimum. In studies using animal models, it was proven that elemental iron is 30-150 times less toxic in comparison to soluble iron salts. The possibility to reduce the dose of such iron to a minimum causes that - in spite of the fact that participation of gastric juices in the transformation of elemental iron to its ionic form - disturbances in the management of gastric juices playing a key role in nutritional processes do not occur.

The fluid form of the composition according to the invention offers a possibility to select the preparation dose individually, by a simple recalculation of the preparation amount per one kilogram of body weight. Such a product fully eliminates the disadvantages of the threshold dosing characteristic for solid formulation and those connected with a risk of overdose, which may lead to poisoning or intensification of side effects, or receiving a dose not sufficient to satisfy the increased demand for iron.

The application of elemental iron with a narrow range of particle sizes, defined according to the invention, leads to very high bioavailability in the human organism and reduces the effect of a high concentration of unused iron ions. This fact, when connected with slow absorption from the digestive tract, causes that elemental iron is liberated gradually and linearly. As a result, toxicity and the intensity of side effects are reduced, and simultaneously, the time required for assimilation of the proper amount of ions is prolonged, as is the time for medical intervention in case of accidental overdosing of the preparation.

Vehicles of the composition according to the invention are characterised by a total lack of reactivity with the iron source, and they do not have oxidative properties. Thanks to this fact, both iron and the vehicle stably maintain their physico-chemical properties in their interaction. Thus, not only water as a vehicle, but also the necessity to use any substances preserving and stabilising the composition were eliminated. Moreover, part of the mentioned oils, particularly MCT oil, exhibit antibacterial and antifungal properties, resulting from their specific chemical structure, additionally contributing to the high stability of the composition. It should be emphasised that application of a mixture of medium-chain oils as a vehicle, particularly a complex of oils from the MCT group (mixture of medium-chain triglycerides), additionally affects the organism favourably, considering the positive effect of the vehicle itself. Moreover, the composition according to the invention provides a neutral taste and aroma.

The preferable profile of iron release from the preparation according to the invention allowed for determining an optimum quantitative ratio of the active component to the vehicle in the ingested dose of the final product. Safety of use and the organoleptic properties of the vehicle (taste, aroma, colour) play an important role, particularly in the case of products used in paediatrics. The amount of the vehicle used in relation to the active component is also not insignificant in the case of preparations for children and infants, as well as for pregnant women and women planning pregnancy.

The strictly defined size of used particles of elemental iron also allowed for minimising the risk connected with induction of pathologic activity of bacterial strains present in the physiological flora of intestines by providing exogenous iron in amounts exceeding the assimilation capacity of the organism.

The combination of components of the composition according to the invention solves the problems existing up to the present and constituting a common feature of all iron-containing preparations, thanks to the fact that the combination has the following properties:
- possibility of linear, continuous dosing adjusted to the needs of the individual patient;
- simple dose determination;
- high iron bioavailability;
- elimination of the necessity to use additional stabilisers or preservatives;
- stability over time;
- elimination of the risk of formation of an ionised form with an oxidation state which is hard to determine;
- elimination of the necessity to use modified formulations in order to improve bioavailability and protect the organism from high doses of iron;
- significant reduction of the intensity of side effects;
- reduction of the risk of an accidental or intentional overdosing of the preparation;
- elimination of metallic taste in the mouth;
- elimination of teeth staining, which is characteristic for liquid products with iron ions;
- lack of unfavourable features characteristic for a preparation having the form of a gel: hard and time-consuming restoration of homogeneity of the preparation after sedimentation of iron particles, inconvenient application of the dose connected with high viscosity of the preparation and tendency of the gel to adhere to mucous membranes of the oesophagus and initial sections of the digestive system.

These features make the composition according to the invention a unique and technologically advanced, up-to-date preparation supplementing iron deficiencies or satisfying an increased demand for iron in the human organism. Thanks to the lack of stabilisers and preservatives, the preparation according to the invention may be used by children and infants, as well as pregnant women. The composition is also useful in elimination of the so-called "coffee effect" in persons drinking large quantities of this beverage.

The subject of the invention is described in more detail in the following examples. Example 1.

A composition consisting of: elemental iron with a medium fineness of D₅₀=8.5 micrometres in the amount of 2.958 g and a mixture of medium-chain C6-C14 triglycerides, known as MCT oil, in the amount of 95.600 g was prepared in the following manner.

The total weighed amount of iron was added to a tank containing 5% of the total amount of oil. The content was stirred vigorously during dosing, at a rate of at least 50 rpm, and the powder was added in batches, in order to avoid possible agglomeration of the material. Stirring was continued for 15 minutes after dosing of the powder. The obtained suspension was completed with the remaining oil and mixed for 30 minutes at a rate of at least 50 rpm. Mixing was continued for the entire duration of the process, and the whole process was carried out at room temperature.

### Example 2.

A composition consisting of: elemental iron with a medium fineness of D₅₀=8.5 micrometres in the amount of 15.915 g. and MCT oil in the amount of 85.600 g was prepared in the following manner.

The total weighed amount of iron was added to a tank with the total amount of the oil. The content was stirred vigorously during dosing, at a rate of at least 50 rpm, and the powder was added in batches, in order to avoid possible agglomeration of the material. After dosing of the powder, the mixture was stirred for 25 minutes more. The mixing process was carried out at room temperature.

### Example 3.

A composition consisting of: elemental iron with a medium fineness of D₅₀=8.5 micrometres in the amount of 2.958 g, tocopherol in the amount of 0.050 g and glycerol in the amount of 95.600 g was prepared in the following manner.

Tocopherol was added to a tank containing 5% of the total amount of glycerol, and then the total weighed amount of iron was added. The content was stirred vigorously during dosing, at a rate of at least 50 rpm, and the powder was added in batches, in order to avoid possible agglomeration of the material. Stirring continued for 20 minutes after dosing of the powder. The obtained suspension was completed with the remaining glycerol and stirred vigorously for no less than 30 minutes at a rate of at least 50 rpm. The process was carried out at room temperature.

### Example 4.

A composition consisting of: elemental iron with a medium fineness of D₅₀=8.5 micrometres in the amount of 2.958 g, tocopherol in the amount of 0.050 g, MCT oil in the amount of 60.000g, olive oil in the amount of 35.500 g, vitamin B₁₂ (0.1% solution in glycerol) in the amount of 0.100 g was prepared in the following manner.

Tocopherol was added to a tank containing 5% of the total amount of MCT, and then the total weighed amount of iron was added. The content was stirred vigorously during dosing, at a rate of at least 50 rpm, and the powder was added in batches, in order to avoid possible agglomeration of the material. Stirring continued for 15 minutes after dosing of the powder. A weighed amount of olive oil was then added, and the contents of the tank were mixed for another 15 minutes. The obtained suspension was completed with the remaining MCT oil and stirred vigorously for no less than 30 minutes at a rate of at least 50 rpm. The entire process was carried at room temperature.

### Example 5.

Studies *in vitro* on the reaction rate of dissolution of elemental iron in 0.1 M hydrochloric acid were carried out. The concentration of iron ions in the samples was determined using the spectrophotometric method by measurement of absorbance at a wavelength of 511 nm in relation to a coloured indicator - phenanthroline (according to the literature data, this value corresponds to maximum absorbance for an iron(II)-phenanthroline complex).

In the study, elemental iron (carbonyl iron - powder) was used with particle size medians of: 5.2 µm (sample A), 9.5 µm (sample B) and 8.5 µm (sample C). Characteristics of the iron particle sizes is shown in Table 1.

**Table 1. Characteristics of iron particle sizes.**

| Sample designation | Particle size distribution median D₅₀ [µm] | D₁₀ [µm] | D₉₀ [µm] |
|---|---|---|---|
| A | 5.2 | 3.5 | 9.2 |
| B | 9.5 | 3.6 | 17.8 |
| C | 8.5 | 2.7 | 13.0 |

10.5 mg of each sample (A, B, C) was weighed, 5 ml 0.1 M of hydrochloric acid at 37°C was added to each of the weigh-outs, up to a concentration of 2.1 mg/ml (the acid was thermostated earlier in an ultrasonic bath). The samples were kept in an ultrasonic washer at 37°C for the entire period of measurements, and 10 µl of the sample in the form of a suspension was collected after a specified time. Before collection, the sample was shaken in order to obtain a homogeneous concentration throughout the entire volume, and then iron was isolated from the solution using a neodymium magnet in order to remove non-ionised iron particles which might still be reacting during the absorbance measurement and disturb the results. The measurements were carried out until a constant concentration of iron(II) ions was obtained - a plateau in the curve of concentration of iron(II) ions in the sample vs. time. Portions of the investigated solution (10 µl each) containing iron(II) ions in hydrochloric acid were transferred to cells containing a solution with the following composition reported in Table 2.

**Table 2. Composition of the sample in the measurement cell before iron(II) ions were added.**

| **Composition of the sample** | **µl** |
|---|---|
| water | 1720 |
| sodium citrate 10% w/w | 20 |
| hydroxylamine hydrochloride 10% w/w | 40 |
| phenanthroline (0.25% w/w in 0.1 M HCl) | 200 |

The absorption spectrum was recorded immediately after a portion of the solution containing iron(II) ions was added to the cell containing other reagents. The spectra were recorded in the range from 360 nm to 700 nm, at a resolution of 1 nm, using a Perkin Elmer Lambda 650 UV-Vis spectrophotometer. For the wavelength of 511 nm, a maximum of absorbance was observed - according to data in literature, this corresponds to absorbance of the iron(II)-phenanthroline complex. Absorbance measurements were carried out at the maximum corresponding to the wavelength of 511 nm. The obtained absorbance values were used for calculations of concentration of iron(II) ions in the sample, based on the analytical curve and taking into account the dilution (the measurement conditions were selected so as all obtained absorbance values were in the range of linearity of the calibration curve). A dependence of changes in the concentration of iron(II) ions in the sample *vs.* time was plotted.

Iron was liberated most rapidly from sample A - maximum absorbance was already obtained after 15 minutes. Sample C dissolved relatively slower than sample A - maximum absorbance was obtained after 20 min. For sample B, a linear dependence of iron release was obtained over time up to 45 min. Maximum absorbance was obtained after 60 min.

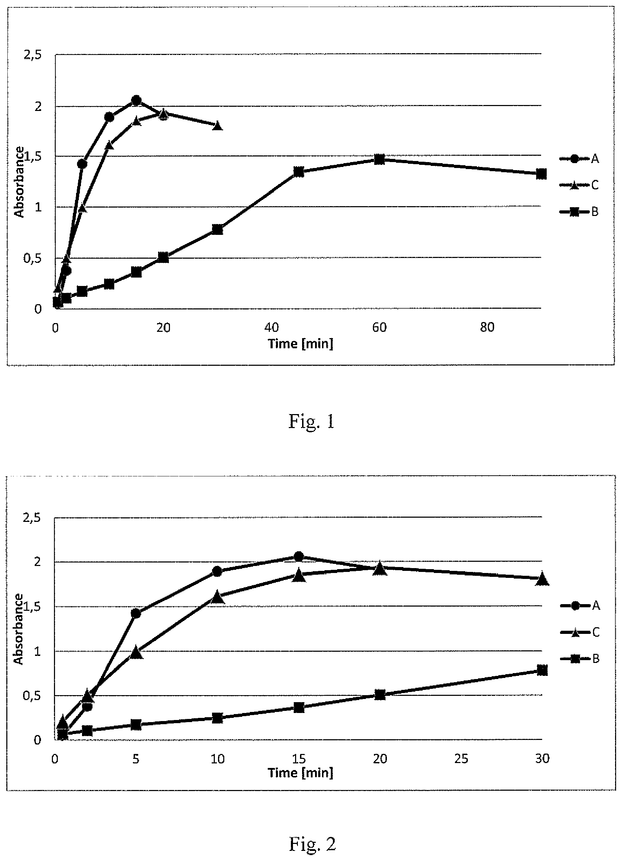

The results are gathered in the graphs: Fig. 1 and Fig. 2 show absorbance of the studied samples A, B and C *vs*. time at a concentration of 2.1 mg/ml. Fig. 2 shows a detailed comparison of samples A, B and C for a concentration of 2.1 mg/ml in order to illustrate precisely the essence of the invention.

As can be seen in Fig. 1 and Fig. 2, iron particles from the size range of 5-8.5 micrometres (calculated as a D₅₀ median) take on an exponential character of the reaction, while a slight increase in the particle size beyond the threshold of 8.5 micrometres (in the study - median of 9.5 micrometres) already causes a transformation of this dependence into a linear form and has a further tendency to behave according to zero-order kinetics.

### Example 6.

A study on pharmaceutical preparations developed based on A and C iron particles was then carried out using the same analytical method. A preparation based on sample A particles was composed according to the description disclosed in Patent Application No. WO 2010/146155A1, while a preparation based on sample C particles was prepared according to the description being the subject of the invention.

**Table 3. Composition of the formulation of the pharmaceutical products based on particles A and C.**

| Product designation | Elemental iron | MCT | Colloidal silica | Aluminium oxide |
|---|---|---|---|---|
| A | 25% | Ad 100 g | 0.625g | 0.125g |
| C | 1.52% | Ad 100 g | - | - |

Before collection of a weigh-out of preparation C, homogenisation of the sample was carried out - shaking for 5 min in a homogeniser (amplitude = 70%, cycle = 0.5) in order to obtain a uniform mixture. A weigh-out of iron oil suspension (carbonyl iron in MCT, 1.52%, D₅₀ = 8.5 µm) - preparation with a weight of 668.7 mg - was dissolved in 0.1 M hydrochloric acid up to a concentration of 2.1 mg/ml. A series of measurements was carried out as in the case of Example 5. A "linear" dependence of concentration of iron(II) ions on the reaction time was obtained for 5-45 min for the maximum at 511 nm (R2=0.999).

Before collection of a weigh-out of preparation A, homogenisation of the sample was carried out - shaking for 5 min in a homogeniser (amplitude = 70%, cycle = 0.5) in order to obtain a uniform mixture. A weigh-out of iron oil suspension (elemental iron, 25%, D₅₀ = 5.2 µm) - preparation with a weight of 42 mg - was dissolved in 0.1 M hydrochloric acid up to a concentration of 2.1 mg/ml. A series of measurements was carried out as in the case of Example 5.

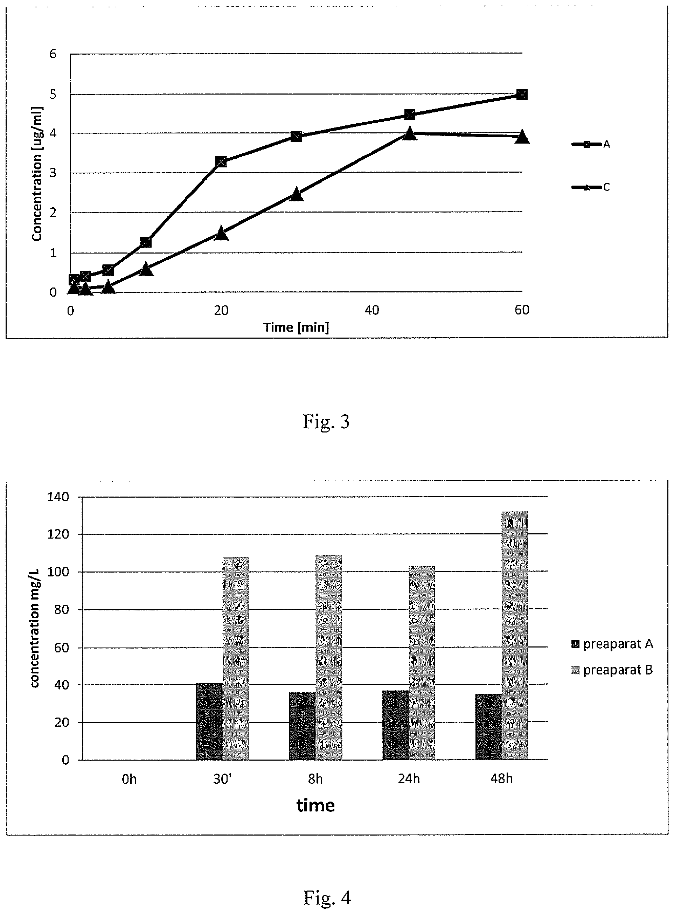

Comparison of the compositions is shown in Table 3, and Fig. 3 shows a comparison of the release rate of iron ions from pharmaceutical preparations based on elemental iron vs. time.

Pharmaceutical preparations made using the particles investigated in Example 5 confirm the tendencies of the release characteristics. Preparation A, in the form of a gel based on MCT oil and particles with a size of 5.2 micrometres (median D₅₀), exhibits a tendency for exponential release of iron ions. Preparation C, containing iron particles with a size of 8.5 micrometres, leads to linear kinetics, and the maximum concentration is obtained in the time corresponding to gastric passage.

### Example 6.

An *in vitro* study showing the differences in the amounts of released iron and iron absorbed by pathogenic strains was carried out using an M9 minimal medium devoid of ion ions, or TSB (tryptic soy broth). Standard strains of *E. coli, Klebsiella pneumoniae* were grown in the M9 minimal medium at 37°C +/-1°C for 24 h. Standard strains of *Enterococcus faecalis, Staphylococcus aureus* with an MRSA mechanism were grown in the TSB (tryptic soy broth) medium at 37°C +/-1°C for 24 h. After this time, the standard strain culture was centrifuged (2000 rpm, 10 min), the supernatant was decanted, and the sediment of bacterial cells that remained on the bottom was washed with 10 ml of the corresponding medium, M9 or TSB, and then centrifuged once again. The procedure was repeated twice. After washing of the cellular deposit, 10 ml of the corresponding medium, M9 or TSB, was added and thoroughly vortexed. The so-prepared pure culture was inoculated on a Columbia agar medium (*Enterococcus faecalis, Staphylococcus aureus*) or McConkey agar (*E. coli, Klebsiella pneumoniae*) in order to determine the initial number of colony forming units (cfu/ml) (by the 10-fold dilutions method), and then they were used for further tests. The so-prepared culture was diluted up to a suspension with a density of 10²/10³ cfu/ml in the corresponding medium (TSB or M9). 300 µl of the suspension were placed in an Ependorff test tube, and 300 µl of the corresponding sample A or B were added. For each strain, 2 Ependorff test tubes for sample A and 2 Ependorff test tubes for sample B (the first one for the iron concentration measurement, the second one for the cfu/ml and OD measurements) were prepared in this way. 2 control samples without cultures were prepared in the same way: by adding 300 µl of sample A or B to 300 µl of M9 medium. All of the above were incubated at 37°C +/-+/- 1°C, then the measurements were carried out after times of: 30 min, 8 h, 24 h, 48 h, using only the aqueous phase. The aqueous phase was centrifuged as a whole, and the cellular pellet (in order to investigate the amount of iron absorbed by the cells) and the remaining supernatant were used for the test separately. In order to free iron from the cellular pellet, the latter was incubated at 60°C for 3 h and then disintegrated using glass beads. Inductively coupled plasma mass spectrometry (ICP-MS) using an ELAN 6100 spectrometer (Perkin Elmer, USA) was used for the measurement of the level of iron ions. Next, the samples (pellet and supernatant) were mineralised by adding 1.2 ml of ultrapure nitric(V) acid, then 100 µl were collected and diluted to 5 ml with distilled water. For the same times, OD was also measured, and a quantitative culture was carried out by the serial dilution method, and simultaneously, positive control was also carried out for the individual strains. The obtained results of concentration of the released iron for samples A and B are shown in Table 4 and in Fig. 4. Concentrations of iron absorbed by the pathogenic bacteria are shown in Tables 5, 6.

**Table 4. Amount of released iron for samples A and B.**

| Time | Preparation A | Preparation B |
|---|---|---|
| | mg/L | mg/L |
| 30' | 41.53 | 108.68 |
| 8 h | 36.45 | 109.29 |
| 24 h | 37.85 | 103.96 |
| 48 h | 35.63 | 132.13 |

**Table 5. Concentration of iron absorbed by strains: S. aureus ATCC -BAA-36 and E. faecalis ATCC 2912 for samples A and B.**

| Time | *S. aureus* ATCC -BAA-36 strain | | *E. faecalis* ATCC 2912 strain | |
|---|---|---|---|---|
| | Concentration of iron absorbed from preparation A, mg/0.3 ml | Concentration of iron absorbed from preparation B, mg/0.3 ml | Concentration of iron absorbed from preparation A, mg/0.3 ml | Concentration of iron absorbed from preparation B, mg/0.3 ml |
| 0 h | 0 | 0 | 0 | 0 |
| 30' | 0.03 | 0.05 | 0.018 | 0.05 |
| 8 h | 0.04 | 0.12 | 0.029 | 0.06 |
| 24 h | 0.05 | 0.159 | 0.04 | 0.54 |
| 48 h | 0.122 | 0.157 | 0.09 | 0.98 |

**Table 6. Concentration of iron absorbed by strains: E. coli ATCC25922 and K. pneumoniae BAA-1705 for samples A and B.**

| Time | *E. coli* ATCC25922 strain | | *K. pneumoniae* BAA-1705 strain | |
|---|---|---|---|---|
| | Concentration of iron absorbed from preparation A, mg/0.3 ml | Concentration of iron absorbed from preparation B, mg/0.3 ml | Concentration of iron absorbed from preparation A, mg/0.3 ml | Concentration of iron absorbed from preparation B, mg/0.3 ml |
| 0 h | 0 | 0 | 0 | 0 |
| 30' | 0.016 | 0.04 | 0.013 | 0.029 |
| 8 h | 0.019 | 2.88 | 0.023 | 0.026 |
| 24 h | 0.0155 | 2.81 | 0.015 | 0.02 |
| 48 h | 0.018 | 0.01 | 0.025 | 0.028 |

## Claims

1. A fluid composition containing a source of iron in non-ionic form and the vehicle, **characterised in that** it consists of elemental iron with average particle size D₅₀ in the range from 7 to 10 micrometres in an amount from 0.1% to 15.0% w/w, and a vehicle selected from glycerol and/or a vegetable oil and/or fish oil in an amount from 85% to 99.9% w/w., individually or in a mixture.

2. A composition according to claim 1, **characterised in that** elemental iron has a particle size distribution with the following characteristics: 7µm< D₅₀<10 µm and D₉₀<15 µm.

3. A composition according to claim 1, **characterised in that** as an oil, it contains: triglycerides of medium-chain fatty acids with a chain length from C6 to C14, individually or in a mixture, glycerol, maize oil, rapeseed oil, linseed oil, untreated coconut oil, fish-liver oil, cod-liver oil or other fish oils, olive oil, or any mixtures thereof.

4. A composition according to claim 3, **characterised in that** it contains a mixture of triglycerides of medium-chain fatty acids, in which the C8-C10 chains constitute at least 90% w/w.

5. A composition according to claim 1, **characterised in that** it additionally contains at least one active component selected from a group consisting of: a mixture of tocopherols, α-tocopherol, organic compounds from the retinoid group, retinol, vitamin K, D vitamins, natural organic carotenoids, vitamin C, vitamins of the B group.

6. A composition according to claim 1, **characterised in that** it additionally contains components from the group: probiotic bacteria, non-pathogenic pure culture yeast, arabinogalactan, inulin.

7. A composition according to claim 5 or 6, **characterised in that** the additional active component contained in the composition is in an amount from 0.001 to 10% w/w.

8. A composition according to claim 1, **characterised in that** it additionally contains at least one pharmaceutically acceptable auxiliary substance.

9. A composition according to claim 8, **characterised in that** the auxiliary substance contained in the composition is in an amount from 1% to 10% w/w.

10. A fluid composition according to claims 1-9 for use in iron deficiencies supplementation.

11. A fluid composition according to claims 1-9 for use to satisfy an increased demand for iron in mammals, including humans.

## Patentansprüche

1. Ein flüssiges Gemisch, das eine Quelle von Eisen in einer nichtionischen Form sowie einen Trägerstoff enthält, **dadurch gekennzeichnet, dass** es aus elementarem Eisen mit einer durchschnittlichen Teilchengröße D₅₀ im Bereich von 7 bis 10 Mikrometern in einer Menge von 0,1 bis 15,0 % G/G und einen Trägerstoff, ausgewählt aus Glycerin und/oder Pflanzenöl und/oder Fischöl in einer Menge von 85 bis 99,9 % G/G, einzeln oder gemischt, besteht.

2. Ein Gemisch nach Anspruch 1, **dadurch gekennzeichnet, dass** elementares Eisen eine Teilchengrößenverteilung mit den folgenden Eigenschaften aufweist: 7 µm< D₅₀<10 µm und D₉₀<15 µm.

3. Ein Gemisch nach Anspruch 1, **dadurch gekennzeichnet, dass** es als ein Ölgemisch folgende Bestandteile enthält: Triglyceride mittelkettiger Fettsäuren mit einer Kettenlänge von C6 bis C14, einzeln oder gemischt, Glycerin, Maisöl, Rapsöl, Leinöl, unbehandeltes Kokosöl, Fischleberöl, Dorschleberöl oder andere Fischöle, Olivenöl oder beliebige Mischungen davon.

4. Ein Gemisch nach Anspruch 3, **dadurch gekennzeichnet, dass** es eine Mischung von Triglyceriden mittelkettiger Fettsäuren enthält, in der die C8-C10-Ketten mindestens 90 % G/G ausmachen.

5. Ein Gemisch nach Anspruch 1, **dadurch gekennzeichnet, dass** es zusätzlich mindestens eine aktive Komponente enthält, die aus einer Gruppe folgender Zutaten bestehen: einer Mischung von Tocopherolen, α-Tocopherol, organischen Verbindungen aus der Gruppe der Retinoide, Retinol, Vitamin K, D-Vitamine, natürlichen organischen Carotinoiden, Vitamin C, Vitaminen der B-Gruppe.

6. Ein Gemisch nach Anspruch 1, **dadurch gekennzeichnet, dass** es zusätzlich Komponenten aus der folgenden Gruppe: probiotische Bakterien, nicht-pathogene Reinzuchthefe, Arabinogalactan, Inulin enthält.

7. Ein Gemisch nach Anspruch 5 oder 6, **dadurch gekennzeichnet, dass** die zusätzliche aktive Komponente, die im Gemisch enthalten ist, in einer Menge von 0,001 bis 10% G/G vorliegt.

8. Ein Gemisch nach Anspruch 1, **dadurch gekennzeichnet, dass** es zusätzlich mindestens einen pharmazeutisch zugelassenen Hilfsstoff enthält.

9. Ein Gemisch nach Anspruch 8, **dadurch gekennzeichnet, dass** die im Gemisch enthaltene Hilfssubstanz in einer Menge von 1% bis 10% G/G vorliegt.

10. Ein flüssiges Gemisch nach Ansprüchen 1-9 zur Verwendung als Nahrungsergänzungsmittel bei Eisenmangel.

11. Ein flüssiges Gemisch nach Ansprüchen 1-9 zur Verwendung als Nahrungsergänzungsmittel bei erhöhtem Eisenbedarf bei Säugetieren, einschließlich Menschen.

## Revendications

1. Composition fluide contenant une source de fer sous forme non ionique et le véhicule, **caractérisée en ce qu'**elle est constituée de fer élémentaire avec une taille moyenne de particules D₅₀ dans la gamme de 7 à 10 micromètres en une quantité de 0,1% à 15,0% en poids/poids, et un véhicule choisi parmi le glycérol et/ou une huile végétale et/ou une huile de poisson en une quantité de 85% à 99,9% poids/poids, individuellement ou en mélange.

2. Composition selon la revendication 1, **caractérisée en ce que** le fer élémentaire a une distribution granulométrique avec les caractéristiques suivantes: 7 µm< D₅₀<10 µm et D₉₀<15 µm.

3. Composition selon la revendication 1, **caractérisée en ce qu** 'en tant qu'huile, elle contient: des triglycérides d'acides gras à chaîne moyenne avec une longueur de chaîne de C₆ à C₁₄, individuellement ou en mélange, glycérol, huile de maïs, huile de colza, huile de lin, l'huile de coco non traitée, l'huile de foie de poisson, l'huile de foie de morue ou d'autres huiles de poisson, l'huile d'olive ou tout mélange de celles-ci.

4. Composition selon la revendication 3, **caractérisée en ce qu'**elle contient un mélange de triglycérides d'acides gras à chaîne moyenne, dans lequel les chaînes C₈-C₁₀ constituent au moins 90% poids/poids.

5. Composition selon la revendication 1, **caractérisée en ce qu'**elle contient en outre au moins un composant actif choisi dans un groupe constitué de: un mélange de tocophérols, α-tocophérol, de composés organiques du groupe rétinoïde, rétinol, vitamine K, vitamines D, naturelles caroténoïdes organiques, vitamine C, vitamines du groupe B.

6. Composition selon la revendication 1, **caractérisée en ce qu'**elle contient en outre des composants du groupe: bactéries probiotiques, levure de culture pure non pathogène, arabinogalactane, inuline.

7. Composition selon la revendication 5 ou 6, **caractérisée en ce que** le composant actif supplémentaire contenu dans la composition est en une quantité de 0,001 à 10% en poids/poids.

8. Composition selon la revendication 1, **caractérisée en ce qu'**elle contient en outre au moins une substance auxiliaire pharmaceutiquement acceptable.

9. Composition selon la revendication 8, **caractérisée en ce que** la substance auxiliaire contenue dans la composition est en une quantité de 1% à 10% poids/poids.

10. Composition fluide selon les revendications 1 à 9 à utiliser dans la supplémentation en carences en fer.

11. Composition fluide selon les revendications 1 à 9 à utiliser pour satisfaire une demande accrue de fer chez les mammifères, y compris les humains.
